(19) Europäisches Patentamt
European Patent Office
Office européen des brevets

(11)   **EP 3 871 518 B1**

(12)   **EUROPEAN PATENT SPECIFICATION**

(45) Date of publication and mention
of the grant of the patent:
**21.12.2022 Bulletin 2022/51**

(21) Application number: **21159229.0**

(22) Date of filing: **25.02.2021**

(51) International Patent Classification (IPC):
**A24F 40/30** (2020.01)    **A24F 40/50** (2020.01)
**A24F 40/57** (2020.01)

(52) Cooperative Patent Classification (CPC):
**A24F 40/57; A24F 40/30; A24F 40/50;** A24F 40/10;
A24F 40/20; A24F 40/46

(54) **POWER SUPPLY UNIT FOR AEROSOL INHALER AND AEROSOL INHALER**

STROMVERSORGUNGSEINHEIT FÜR AEROSOLINHALATOR SOWIE AEROSOLINHALATOR

UNITÉ D'ALIMENTATION ÉLECTRIQUE POUR INHALATEUR AÉROSOL ET INHALATEUR AÉROSOL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.02.2020 JP 2020029901**

(43) Date of publication of application:
**01.09.2021 Bulletin 2021/35**

(73) Proprietor: **Japan Tobacco Inc.**
**Tokyo 105-6927 (JP)**

(72) Inventors:
• **Nakano, Takuma**
**Tokyo, 130-0003 (JP)**
• **Kaihatsu, Yutaka**
**Tokyo, 130-0003 (JP)**
• **Marubashi, Keiji**
**Tokyo, 130-0003 (JP)**
• **Fujita, Hajime**
**Tokyo, 130-0003 (JP)**

(74) Representative: **Hoffmann Eitle**
**Patent- und Rechtsanwälte PartmbB**
**Arabellastraße 30**
**81925 München (DE)**

(56) References cited:
**EP-A1- 3 066 942    WO-A1-2020/027448**

EP 3 871 518 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a power supply unit for an aerosol inhaler and an aerosol inhaler.

BACKGROUND ART

JP 2017-511703 T, WO 2019/017654, and WO 2018/020619 disclose an apparatus that can add a flavor component contained in a flavor source to an aerosol by passing the aerosol generated by heating a liquid through the flavor source, and cause a user to suck the aerosol containing the flavor component.

Apparatuses disclosed in JP 2017-511703 T and WO 2019/017654 each include a heater that heats a liquid for aerosol generation and a heater that heats a flavor source.

WO 2018/020619 discloses that control of boosting an output voltage of a battery is performed such that an amount of aerosol generation does not decrease in conjunction with a decrease in the output voltage of the battery.

It is important that an aerosol inhaler can provide a large amount of a flavor component to the user in a stable manner in order to increase a commercial value. JP 2017-511703 T, WO 2019/017654, and WO 2018/020619 do not consider providing the flavor component to the user in a stable manner.

It is an object of the present invention to increase the commercial value of the aerosol inhaler.

WO 2020/027448 A1 describes an aerosol generating device comprising: a heater for heating a cigarette accommodated in the device; a battery for supplying power to the heater; and a control unit for controlling power supplied to the heater such that a temperature of the heater is equal to or higher than a reference temperature which is set so as to suitably generate an aerosol from the cigarette, and controlling power supplied to the heater by setting a target temperature of the heater so as to maintain the temperature of the heater at the reference temperature, wherein the control unit adjusts the target temperature to compensate for the temperature of the heater that decreases below the reference temperature.

The power supply unit for an aerosol inhaler according to WO2020/027448A1 causes an aerosol generated from an aerosol source heated by a first load to pass through a flavor source heated by a second load to add a flavor component of the flavor source to the aerosol, and it comprises: a power supply configured to be dischargeable to the first load and the second load; and a processing device, wherein the processing device is configured to control discharging from the power supply to the first load in response to a signal from a first sensor configured to output the signal indicating an aerosol generation request, wherein the processing device is configured to control discharging from the power supply to the second load such that a temperature of the flavor source converges to a target temperature based on an output of a second sensor configured to output information on a temperature of the flavor source, and wherein the processing device is configured to increase the target temperature, based on a cumulative time of discharging to the first load in response to the signal.

EP 3 066 942 A1 describes a method of controlling aerosol production in an aerosol-generating device, the device comprising: a heater comprising at least one heating element configured to heat an aerosol-forming substrate; and a power source for providing power to the heating element, comprising the steps of: controlling the power provided to the heating element such that in a first phase power is provided such that the temperature of the heating element increases from an initial temperature to a first temperature, in a second phase power is provided such that the temperature of the heating element drops below the first temperature and in a third phase power is provided such that the temperature of the heating element increases again. Increasing the temperature of the heating element during a final phase of the heating process reduces or prevents the reduction in aerosol delivery over time.

SUMMARY OF INVENTION

According to an aspect of the present invention, there is provided a power supply unit for an aerosol inhaler that causes an aerosol generated from an aerosol source heated by a first load to pass through a flavor source heated by a second load to add a flavor component of the flavor source to the aerosol. The power supply unit includes a power supply configured to be dischargeable to the first load and the second load, and a processing device. The processing device is configured to control discharging from the power supply to the first load in response to a signal from a first sensor configured to output the signal indicating an aerosol generation request. The processing device is configured to control discharging from the power supply to the second load such that a temperature of the flavor source converges to a target temperature based on an output of a second sensor configured to output information on a temperature of the flavor source. The processing device is configured to increase the target temperature such that a period during which the highest target temperature is applied is the longest, based on a cumulative number of times of discharging to the first load in response to the signal or a cumulative time of discharging to the first load in response to the signal.

According to another aspect of the present invention, there is provided an aerosol inhaler as set out in Claim 15.

BRIEF DESCRIPTION OF DRAWINGS

Fig. 1 is a perspective view schematically showing a schematic configuration of an aerosol inhaler.
Fig. 2 is another perspective view of the aerosol inhaler of Fig. 1.
Fig. 3 is a cross-sectional view of the aerosol inhaler of Fig. 1.
Fig. 4 is a perspective view of a power supply unit of the aerosol inhaler of Fig. 1.
Fig. 5 is a schematic diagram showing a hardware configuration of the aerosol inhaler of Fig. 1.
Fig. 6 is a schematic diagram showing a modification of the hardware configuration of the aerosol inhaler of Fig. 1.
Fig. 7 is a diagram showing a specific example of a power supply unit shown in Fig. 5.
Fig. 8 is a diagram showing a specific example of a power supply unit shown in Fig. 6.
Fig. 9 is a diagram showing results obtained by calculating a target temperature of a flavor source such that an amount of a flavor component converges to a target amount, and measurement results of the amount of the flavor component when discharging control to a second load is performed based on the result.
Fig. 10 is a flowchart for illustrating operations of the aerosol inhaler of Fig. 1.
Fig. 11 is a flowchart for illustrating operations of the aerosol inhaler of Fig. 1.
Fig. 12 is a schematic diagram for illustrating a second modification of the operations of the aerosol inhaler.

DESCRIPTION OF EMBODIMENTS

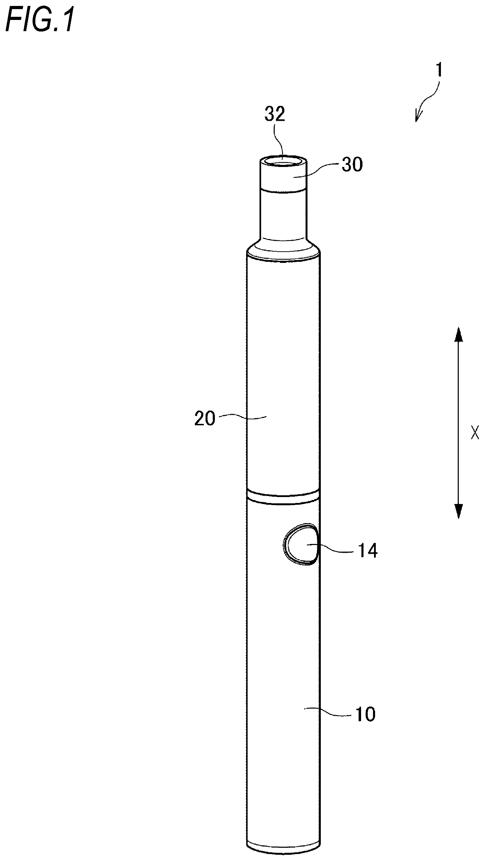

Hereinafter, an aerosol inhaler 1, which is an embodiment of an aerosol inhaler of the present invention, will be described with reference to Figs. 1 to 5.

(Aerosol Inhaler)

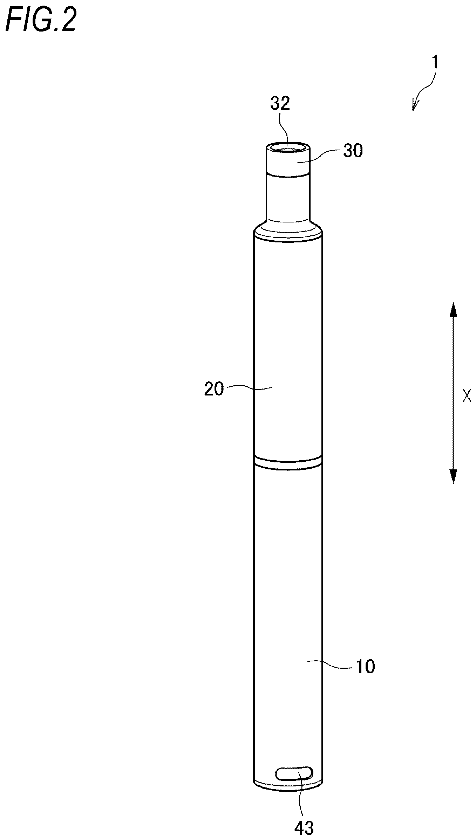

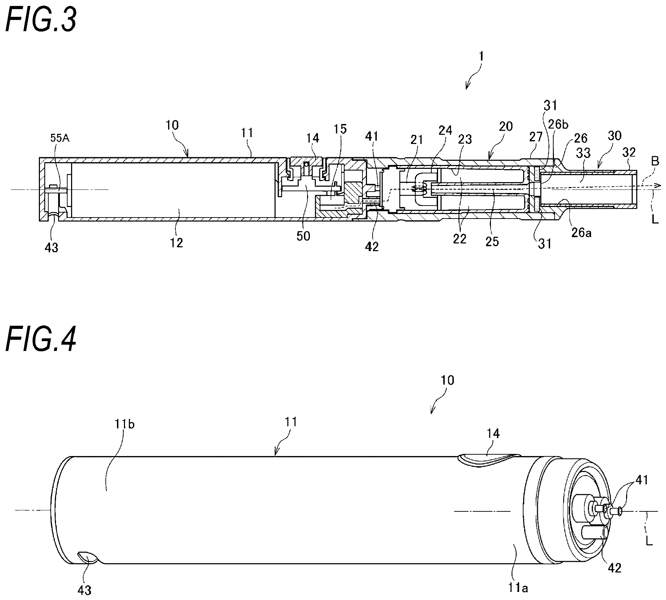

The aerosol inhaler 1 is a device for generating an aerosol to which a flavor component is added without burning and making it possible to suck the aerosol, and has a rod shape that extends along a predetermined direction (hereinafter, referred to as longitudinal direction X) as shown in Figs. 1 and 2. In the aerosol inhaler 1, a power supply unit 10, a first cartridge 20, and a second cartridge 30 are provided in this order along the longitudinal direction X. The first cartridge 20 is attachable to and detachable from (in other words, replaceable with respect to) the power supply unit 10. The second cartridge 30 is attachable to and detachable from (in other words, replaceable with respect to) the first cartridge 20. As shown in Fig. 3, the first cartridge 20 is provided with a first load 21 and a second load 31. As shown in Fig. 1, an overall shape of the aerosol inhaler 1 is not limited to a shape in which the power supply unit 10, the first cartridge 20, and the second cartridge 30 are lined up in a row. As long as the first cartridge 20 and the second cartridge 30 are replaceable with respect to the power supply unit 10, any shape such as a substantial box shape can be adopted. The second cartridge 30 may be attachable to and detachable from (in other words, replaceable with respect to) the power supply unit 10.

(Power Supply Unit)

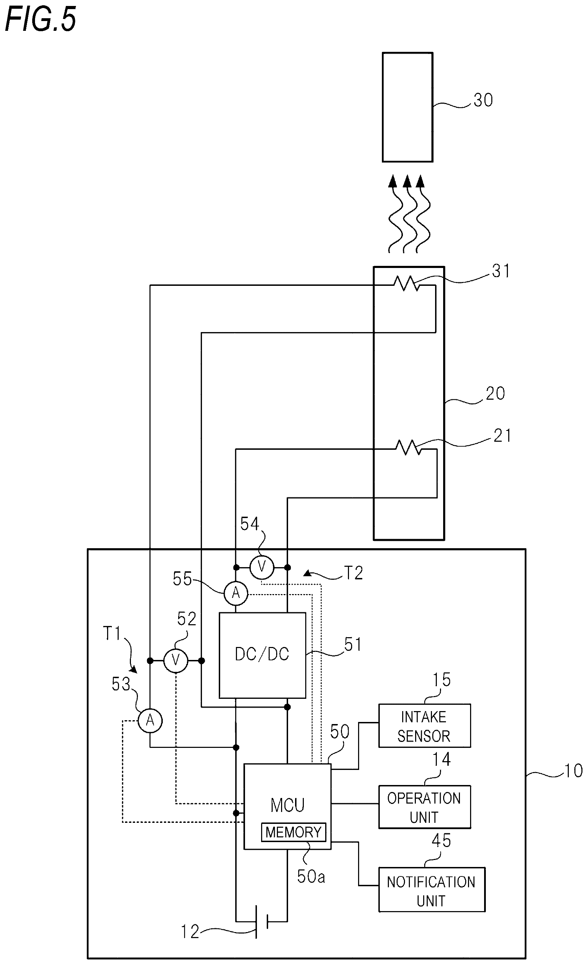

As shown in Figs. 3, 4, and 5, the power supply unit 10 houses a power supply 12, a charging IC 55A, a micro controller unit (MCU) 50, a DC/DC converter 51, an intake sensor 15, a temperature detection element T1 including a voltage sensor 52 and a current sensor 53, and a temperature detection element T2 including a voltage sensor 54 and a current sensor 55 inside a cylindrical power supply unit case 11.
The power supply 12 is a rechargeable secondary battery, an electric double-layer capacitor, or the like, and is preferably a lithium-ion secondary battery. An electrolyte of the power supply 12 may be one of or a combination of a gel-like electrolyte, an electrolytic solution, a solid electrolyte, and an ionic liquid.
As shown in Fig. 5, the MCU 50 is connected to various sensor devices such as the intake sensor 15, the voltage sensor 52, the current sensor 53, the voltage sensor 54, and the current sensor 55, the DC/DC converter 51, an operation unit 14, and a notification unit 45, and performs various kinds of control of the aerosol inhaler 1.
Specifically, the MCU 50 is mainly configured with a processor, and further includes a memory 50a configured with a storage medium such as a random access memory (RAM) required for an operation of the processor and a read only memory (ROM) that stores various pieces of information. Specifically, the processor in the present description is an electric circuit in which circuit elements such as semiconductor elements are combined.
As shown in Fig. 4, discharging terminals 41 are provided on a top portion 11a positioned on one end side of the power supply unit case 11 in the longitudinal direction X (a first cartridge 20 side). The discharging terminal 41 is provided so as to protrude from an upper surface of the top portion 11a toward the first cartridge 20, and can be electrically connected to the first load 21 and the second load 31 of the first cartridge 20.

On the upper surface of the top portion 11a, an air supply unit 42 that supplies air to the first load 21 of the first cartridge 20 is provided in the vicinity of the discharging terminals 41.

A charging terminal 43 that can be electrically connected to an external power supply (not shown) is provided in a bottom portion 11b positioned on the other end side of the power supply unit case 11 in the longitudinal direction X (a side opposite to the first cartridge 20). The charging terminal 43 is provided in a side surface of the bottom portion 11b, and can be connected to, for example, a Universal Serial Bus (USB) terminal, a micro USB terminal, a Lightning (registered trademark) terminal, or the like.

The charging terminal 43 may be a power reception unit that can receive power transmitted from an external power supply in a wireless manner. In such a case, the charging terminal 43 (the power reception unit) may be configured with a power reception coil. A method for wireless power transfer may be an electromagnetic induction type or a magnetic resonance type. Further, the charging terminal 43 may be a power reception unit that can receive power transmitted from an external power supply without contact. As another example, the charging terminal 43 may be connected to the USB terminal, the micro USB terminal, or the Lightning terminal, and may include the power reception unit described above.

The power supply unit case 11 is provided with the operation unit 14 that can be operated by a user in the side surface of the top portion 11a so as to face a side opposite to the charging terminal 43. More specifically, the operation unit 14 and the charging terminal 43 have a point-symmetrical relationship with respect to an intersection between a straight line connecting the operation unit 14 and the charging terminal 43 and a center line of the power supply unit 10 in the longitudinal direction X. The operation unit 14 is configured with a button-type switch, a touch panel, or the like.

As shown in Fig. 3, the intake sensor 15 that detects a puff (suction) operation is provided in the vicinity of the operation unit 14. The power supply unit case 11 is provided with an air intake port (not shown) that takes outside air into the power supply unit case 11. The air intake port may be provided around the operation unit 14 or may be provided around the charging terminal 43.

The intake sensor 15 is configured to output a value of a pressure (an internal pressure) change in the power supply unit 10 caused by suction of the user through a suction port 32 described later. The intake sensor 15 is, for example, a pressure sensor that outputs an output value (for example, a voltage value or a current value) corresponding to an internal pressure that changes according to a flow rate of air sucked from the air intake port toward the suction port 32 (that is, a puff operation of the user). The intake sensor 15 may output an analog value or may output a digital value converted from the analog value.

The intake sensor 15 may incorporate a temperature sensor that detects a temperature of an environment (an outside air temperature) in which the power supply unit 10 is placed in order to compensate for a detected pressure. The intake sensor 15 may be configured with a condenser microphone or the like instead of the pressure sensor.

When a puff operation is performed and an output value of the intake sensor 15 is larger than a threshold, the MCU 50 determines that an aerosol generation request has been made, and then, when the output value of the intake sensor 15 is smaller than the threshold, the MCU 50 determines that the aerosol generation request has been ended. In the aerosol inhaler 1, when a period during which the aerosol generation request is made reaches a first default value $t_{upper}$ (for example, 2.4 seconds) for a purpose of preventing overheating of the first load 21 or the like, it is determined that the aerosol generation request has been ended regardless of an output value of the intake sensor 15. Accordingly, the output value of the intake sensor 15 is used as a signal indicating the aerosol generation request. Therefore, the intake sensor 15 constitutes a sensor that outputs an aerosol generation request.

Instead of the intake sensor 15, the aerosol generation request may be detected based on an operation of the operation unit 14. For example, when the user performs a predetermined operation on the operation unit 14 to start sucking aerosol, the operation unit 14 may be configured to output a signal indicating the aerosol generation request to the MCU 50. In this case, the operation unit 14 constitutes a sensor that outputs an aerosol generation request.

The charging IC 55A is disposed close to the charging terminal 43, and controls charging of power input from the charging terminal 43 to the power supply 12. The charging IC 55A may be disposed in the vicinity of the MCU 50.

(First Cartridge)

As shown in Fig. 3, the first cartridge 20 includes inside a cylindrical cartridge case 27, a reservoir 23 that stores an aerosol source 22, the first load 21 for atomizing the aerosol source 22, a wick 24 that draws the aerosol source from the reservoir 23 to the first load 21, an aerosol flow path 25 in which an aerosol generated by atomizing the aerosol source 22 flows toward the second cartridge 30, an end cap 26 that houses a part of the second cartridge 30, and the second load 31 provided in the end cap 26 and configured to heat the second cartridge 30.

The reservoir 23 is partitioned and formed so as to surround a periphery of the aerosol flow path 25 and stores the aerosol source 22. The reservoir 23 may house a porous body such as a resin web or cotton, and the aerosol source 22 may be impregnated in the porous body. The reservoir 23 may not house the porous body in the resin web or cotton and may only store the aerosol source 22. The aerosol source 22 contains a liquid such as glycerin, propylene glycol, or water.

The wick 24 is a liquid holding member that draws the aerosol source 22 from the reservoir 23 to the first load 21 by using a capillary phenomenon. The wick 24 is formed of, for example, glass fiber or porous ceramic.

The first load 21 atomizes the aerosol source 22 by heating the aerosol source 22 without burning by power supplied from the power supply 12 via the discharging terminals 41. The first load 21 is configured with an electric heating wire (a coil) wound at a predetermined pitch.

The first load 21 may be an element that can generate an aerosol by atomizing the aerosol source 22 by heating the aerosol source 22. The first load 21 is, for example, a heat generation element. Examples of the heat generation element include a heat generation resistor, a ceramic heater, and an induction heating type heater.

As the first load 21, a load in which a temperature and an electric resistance value have a correlation is used. As the first load 21, for example, a load having a positive temperature coefficient (PTC) characteristic in which the electric resistance value increases as the temperature increases is used.

The aerosol flow path 25 is provided on a downstream side of the first load 21 and on a center line L of the power supply unit 10. The end cap 26 includes a cartridge housing portion 26a that houses a part of the second cartridge 30, and a communication path 26b that causes the aerosol flow path 25 and the cartridge housing portion 26a to communicate with each other.

The second load 31 is embedded in the cartridge housing portion 26a. The second load 31 heats the second cartridge 30 (more specifically, a flavor source 33 included herein) housed in the cartridge housing portion 26a by the power supplied from the power supply 12 via the discharging terminals 41. The second load 31 is configured with, for example, an electric heating wire (a coil) wound at a predetermined pitch.

The second load 31 may be any element that can heat the second cartridge 30. The second load 31 is, for example, a heat generation element. Examples of the heat generation element include a heat generation resistor, a ceramic heater, and an induction heating type heater.

As the second load 31, a load in which a temperature and an electric resistance value have a correlation is used. As the second load 31, for example, a load having the PTC characteristic is used.

(Second Cartridge)

The second cartridge 30 stores the flavor source 33. When the second cartridge 30 is heated by the second load 31, the flavor source 33 is heated. The second cartridge 30 is detachably housed in the cartridge housing portion 26a provided in the end cap 26 of the first cartridge 20. An end portion of the second cartridge 30 on a side opposite to the first cartridge 20 side is the suction port 32 for the user. The suction port 32 is not limited to being integrally formed with the second cartridge 30 and may be attachable to and detachable from the second cartridge 30. Accordingly, the suction port 32 is configured separately from the power supply unit 10 and the first cartridge 20, so that the suction port 32 can be kept hygienic.

The second cartridge 30 adds a flavor component to an aerosol by passing, through the flavor source 33, the aerosol generated by atomizing the aerosol source 22 by the first load 21. As a raw material piece that constitutes the flavor source 33, cut tobacco or a molded body obtained by molding a tobacco raw material into a granular shape can be used. The flavor source 33 may be configured with a plant other than the tobacco (for example, mint, Chinese medicine, or herbs). A fragrance such as menthol may be added to the flavor source 33.

In the aerosol inhaler 1, the aerosol source 22 and the flavor source 33 can generate an aerosol to which a flavor component is added. That is, the aerosol source 22 and the flavor source 33 constitute an aerosol generation source that generates the aerosol.

The aerosol generation source of the aerosol inhaler 1 is a portion that the user replaces and uses. This portion is provided to the user, for example, as a set of one first cartridge 20 and one or a plurality of (for example, five) second cartridges 30. Therefore, in the aerosol inhaler 1, a replacement frequency of the power supply unit 10 is lowest, a replacement frequency of the first cartridge 20 is second lowest, and a replacement frequency of the second cartridge 30 is highest. Therefore, it is important to reduce a manufacturing cost of the first cartridge 20 and the second cartridge 30. The first cartridge 20 and the second cartridge 30 may be integrated into one cartridge.

In the aerosol inhaler 1 configured in this way, as indicated by an arrow B in Fig. 3, air that flows in from the intake port (not shown) provided in the power supply unit case 11 passes from the air supply unit 42 to a vicinity of the first load 21 of the first cartridge 20. The first load 21 atomizes the aerosol source 22 drawn from the reservoir 23 by the wick 24. An aerosol generated by atomization flows through the aerosol flow path 25 together with the air that flows in from the intake port, and is supplied to the second cartridge 30 via the communication path 26b. The aerosol supplied to the second cartridge 30 passes through the flavor source 33 to add a flavor component and is supplied to the suction port 32.

The aerosol inhaler 1 is provided with the notification unit 45 that notifies various pieces of information (see Fig. 5). The notification unit 45 may be configured with a light-emitting element, may be configured with a vibration element, or may be configured with a sound output element. The notification unit 45 may be a combination of two or more elements among the light-emitting element, the vibration element, and the sound output element. The notification unit 45 may be provided in any of the power supply unit 10, the first cartridge 20, and the second cartridge 30, but is preferably provided in the power supply unit 10. For example, a periphery of the operation unit 14 is translucent, and is configured to emit light by a light-emitting element such as an LED.

(Details of Power Supply Unit)

As shown in Fig. 5, in a state where the first cartridge 20 is mounted on the power supply unit 10, the DC/DC converter 51 is connected between the first load 21 and the power supply 12. The MCU 50 is connected between the DC/DC converter 51 and the power supply 12. In a state where the first cartridge 20 is mounted on the power supply unit 10, the second load 31 is connected to a connection node between the MCU 50 and the DC/DC converter 51. Accordingly, in the power supply unit 10, in a state where the first cartridge 20 is mounted, the second load 31 and a series circuit of the DC/DC converter 51 and the first load 21 are connected in parallel to the power supply 12.

The DC/DC converter 51 is a boosting circuit that can boost an input voltage, and is configured to be able to supply an input voltage or a voltage obtained by boosting the input voltage to the first load 21. Since power supplied to the first load 21 can be adjusted by the DC/DC converter 51, an amount of the aerosol source 22 to be atomized by the first load 21 can be controlled. As the DC/DC converter 51, for example, a switching regulator that converts an input voltage into a desired output voltage can be used by controlling on/off time of a switching element while monitoring the output voltage. When the switching regulator is used as the DC/DC converter 51, the input voltage can be output as it is without boosting by controlling the switching element.

The processor of the MCU 50 is configured to be able to acquire a temperature of the flavor source 33 in order to control discharging to the second load 31, which will be described later. The processor of the MCU 50 is preferably configured to be able to acquire a temperature of the first load 21. The temperature of the first load 21 can be used to prevent overheating of the first load 21 and the aerosol source 22 and to highly control the amount of the aerosol source 22 to be atomized by the first load 21.

The voltage sensor 52 measures and outputs a value of a voltage applied to the second load 31. The current sensor 53 measures and outputs a value of a current that flows through the second load 31. An output of the voltage sensor 52 and an output of the current sensor 53 are input to the MCU 50. The processor of the MCU 50 acquires a resistance value of the second load 31 based on the output of the voltage sensor 52 and the output of the current sensor 53, and acquires a temperature of the second load 31 according to the resistance value. The temperature of the second load 31 does not exactly coincide with a temperature of the flavor source 33 heated by the second load 31, but can be regarded as substantially the same as the temperature of the flavor source 33. Therefore, the temperature detection element T1 constitutes a temperature detection element for detecting the temperature of the flavor source 33.

If a constant current flows to the second load 31 when the resistance value of the second load 31 is acquired, the current sensor 53 is unnecessary in the temperature detection element T1. Similarly, if a constant voltage is applied to the second load 31 when the resistance value of the second load 31 is acquired, the voltage sensor 52 is unnecessary in the temperature detection element T1.

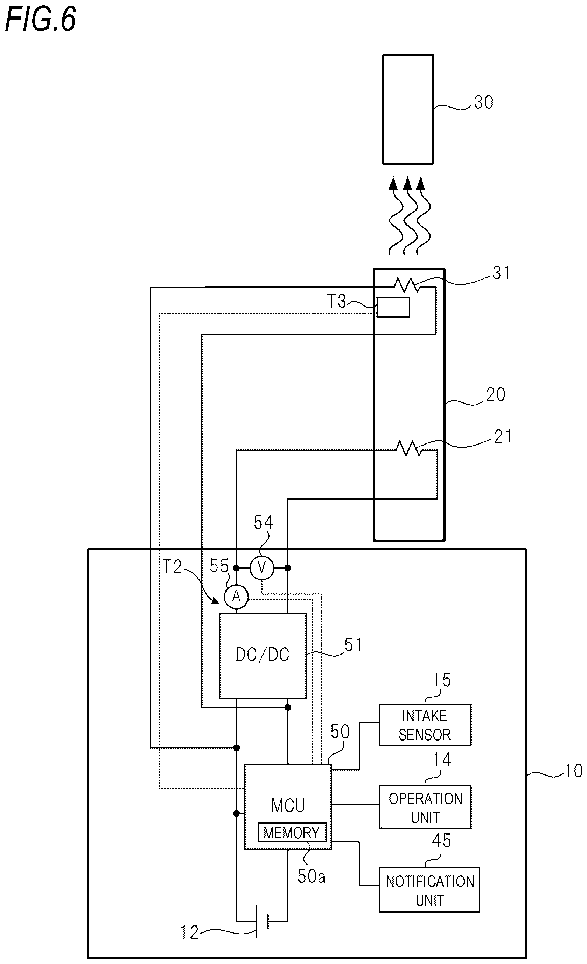

As shown in Fig. 6, instead of the temperature detection element T1, the first cartridge 20 may be provided with a temperature detection element T3 for detecting a temperature of the second cartridge 30. The temperature detection element T3 is configured with, for example, a thermistor disposed in the vicinity of the second cartridge 30. In the configuration of Fig. 6, the processor of the MCU 50 acquires the temperature of the second cartridge 30 (in other words, the flavor source 33) based on an output of the temperature detection element T3.

As shown in Fig. 6, since the temperature of the second cartridge 30 (the flavor source 33) is acquired by using the temperature detection element T3, compared with acquiring the temperature of the flavor source 33 by using the temperature detection element T1 in Fig. 5, the temperature of the flavor source 33 can be more accurately acquired. The temperature detection element T3 may be mounted on the second cartridge 30. According to the configuration shown in Fig. 6 in which the temperature detection element T3 is mounted on the first cartridge 20, a manufacturing cost of the second cartridge 30 having highest replacement frequency in the aerosol inhaler 1 can be reduced.

As shown in Fig. 5, when the temperature of the second cartridge 30 (the flavor source 33) is acquired by using the temperature detection element T1, the temperature detection element T1 can be provided in the power supply unit 10 having lowest replacement frequency in the aerosol inhaler 1. Therefore, manufacturing costs of the first cartridge 20 and the second cartridge 30 can be reduced.

The voltage sensor 54 measures and outputs a value of a voltage applied to the first load 21. The current sensor 55 measures and outputs a value of a current that flows through the first load 21. An output of the voltage sensor 54 and an output of the current sensor 55 are input to the MCU 50. The processor of the MCU 50 acquires a resistance value of the first load 21 based on the output of the voltage sensor 54 and the output of the current sensor 55, and acquires a temperature of the first load 21 according to the resistance value. If a constant current flows to the first load 21 when the resistance value of the first load 21 is acquired, the current sensor 55 is unnecessary in the temperature detection element T2. Similarly, if a constant voltage is applied to the first load 21 when the resistance value of the first load 21 is acquired, the voltage sensor 54 is unnecessary in the temperature detection element T2.

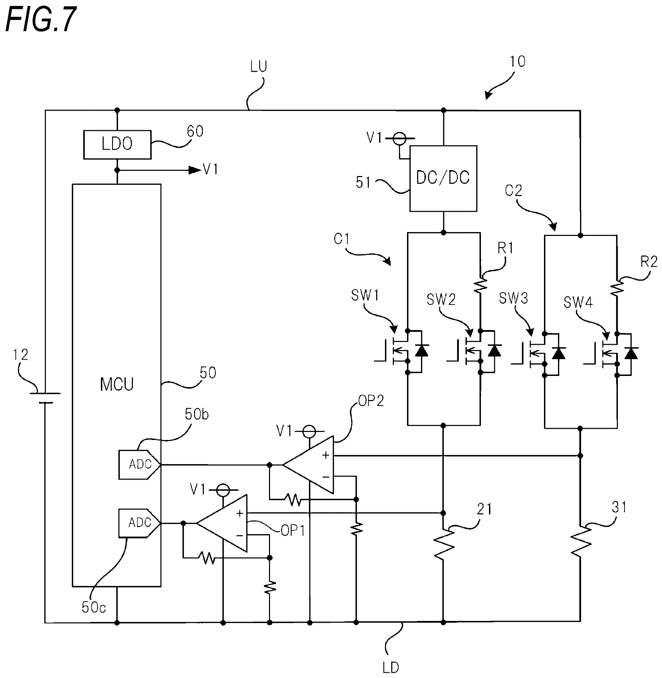

Fig. 7 is a diagram showing a specific example of the power supply unit 10 shown in Fig. 5. Fig. 7 shows a specific example of a configuration in which the temperature detection element T1 does not include the current sensor 53 and the temperature detection element T2 does not include the current sensor 55.

As shown in Fig. 7, the power supply unit 10 includes the power supply 12, the MCU 50, a low drop out (LDO) regulator 60, a switch SW1, a parallel circuit C1 including a series circuit of a resistance element R1 and a switch SW2 connected in parallel to the switch SW1, a switch SW3, a parallel circuit C2 including a series circuit of a resistance element R2 and a switch SW4 connected in parallel to the switch SW3, an operational amplifier OP1 and analogto-digital converter (hereinafter, referred to as ADC) 50c that constitute the voltage sensor 54, and an operational amplifier OP2 and an ADC 50b that constitute the voltage sensor 52.

The resistance element described in the present description may be an element having a fixed electric resistance value, for example, a resistor, a diode, or a transistor. In the example of Fig. 7, the resistance element R1 and the resistance element R2 are resistors.

The switch described in the present description is a switching element such as a transistor that switches between interruption and conduction of a wiring path. In the example of Fig. 7, the switches SW1 to SW4 are transistors.

The LDO regulator 60 is connected to a main positive bus LU connected to a positive electrode of the power supply 12. The MCU 50 is connected to the LDO regulator 60 and a main negative bus LD connected to a negative electrode of the power supply 12. The MCU 50 is also connected to the switches SW1 to SW4, and controls opening and closing of these switches. The LDO regulator 60 reduces a voltage from the power supply 12 and outputs the reduced voltage. An output voltage VI of the LDO regulator 60 is also used as respective operation voltages of the MCU 50, the DC/DC converter 51, the operational amplifier OP1, and the operational amplifier OP2.

The DC/DC converter 51 is connected to the main positive bus LU. The first load 21 is connected to the main negative bus LD. The parallel circuit C1 is connected to the DC/DC converter 51 and the first load 21.

The parallel circuit C2 is connected to the main positive bus LU. The second load 31 is connected to the parallel circuit C2 and the main negative bus LD.

A non-inverting input terminal of the operational amplifier OP1 is connected to a connection node between the parallel circuit C1 and the first load 21. An inverting input terminal of the operational amplifier OP1 is connected to an output terminal of the operational amplifier OP1 and the main negative bus LD via the resistance element.

A non-inverting input terminal of the operational amplifier OP2 is connected to a connection node between the parallel circuit C2 and the second load 31. An inverting input terminal of the operational amplifier OP2 is connected to an output terminal of the operational amplifier OP2 and the main negative bus LD via the resistance element.

The ADC 50c is connected to the output terminal of the operational amplifier OP1. The ADC 50b is connected to the output terminal of the operational amplifier OP2. The ADC 50c and the ADC 50b may be provided at an outside of the MCU 50.

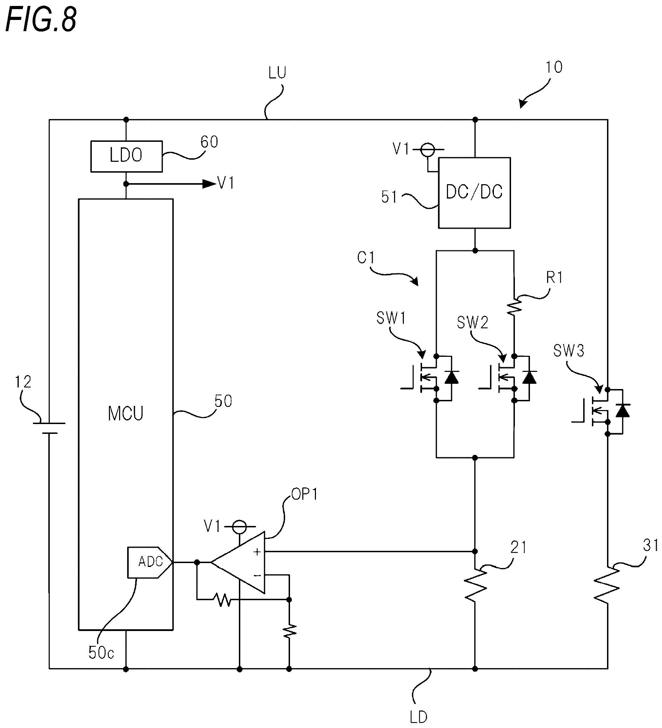

Fig. 8 is a diagram showing a specific example of the power supply unit 10 shown in Fig. 6. Fig. 8 shows a specific example of a configuration in which the temperature detection element T2 does not include the voltage sensor 54. A circuit shown in Fig. 8 has the same configuration as that of Fig. 7 except that the operational amplifier OP2, the ADC 50b, the resistance element R2, and the switch SW4 are eliminated.

(MCU)

Next, functions of the MCU 50 will be described. The MCU 50 includes a temperature detection unit, a power control unit, and a notification control unit as a functional block implemented by executing a program stored in a ROM by the processor.

The temperature detection unit acquires a temperature of the flavor source 33 based on an output of the temperature detection element T1 (or the temperature detection element T3). Further, the temperature detection unit acquires a temperature of the first load 21 based on an output of the temperature detection element T2.

In a case of the circuit example shown in Fig. 7, the temperature detection unit controls the switch SW1, the switch SW3, and the switch SW4 to be in an interruption state, acquires an output value of the ADC 50c (a value of a voltage applied to the first load 21) in a state where the switch SW2 is controlled to be in a conductive state, and acquires a temperature of the first load 21 based on the output value.

The non-inverting input terminal of the operational amplifier OP1 may be connected to a terminal of the resistance element R1 on a DC/DC converter 51 side, and the inverting input terminal of the operational amplifier OP1 may be connected to a terminal of the resistance element R1 on a switch SW2 side. In this case, the temperature detection unit can control the switch SW1, the switch SW3, and the switch SW4 to be in an interruption state, acquire an output value of the ADC 50c (a value of a voltage applied to the resistance element R1) in a state where the switch SW2 is controlled to be in a conductive state, and acquire a temperature of the first load 21 based on the output value.

In the case of the circuit example shown in Fig. 7, the temperature detection unit controls the switch SW1, the switch SW2, and the switch SW3 to be in an interruption state, acquires an output value of the ADC 50b (a value of a voltage applied to the second load 31) in a state where the switch SW4 is controlled to be in a conductive state, and acquires a temperature of the second load 31 as a temperature of the flavor source 33 based on the output value.

The non-inverting input terminal of the operational amplifier OP2 may be connected to a terminal of the resistance element R2 on a main positive bus LU side, and the inverting input terminal of the operational amplifier OP2 may be connected to a terminal of the resistance element R2 on a switch SW4 side. In this case, the temperature detection unit can control the switch SW1, the switch SW2, and the switch SW3 to be in an interruption state, acquire an output value of the ADC 50b (a value of a voltage applied to the resistance element R2) in a state where the switch SW4 is controlled to be in a conductive state, and acquire a temperature of the second load 31 as a temperature of the flavor source 33 based on the output value.

In a case of the circuit example shown in Fig. 8, the temperature detection unit controls the switch SW1 and the switch SW3 to be in an interruption state, acquires an output value of the ADC 50c (a value of a voltage applied to the first load 21) in a state where the switch SW2 is controlled to be in a conductive state, and acquires a temperature of the first load 21 based on the output value.

The notification control unit controls the notification unit 45 so as to notify various pieces of information. For example, the notification control unit controls the notification unit 45 so as to give a notification that prompts replacement of the second cartridge 30 in response to detection of a replacement timing of the second cartridge 30. The notification control unit is not limited to the notification that prompts the replacement of the second cartridge 30, and may give a notification that prompts replacement of the first cartridge 20, a notification that prompts replacement of the power supply 12, a notification that prompts charging of the power supply 12, and the like.

The power control unit controls discharging from the power supply 12 to at least the first load 21 (discharging required for heating a load) of the first load 21 and the second load 31 in response to a signal indicating the aerosol generation request output from the intake sensor 15.

In the case of the circuit example shown in Fig. 7, the power control unit controls the switch SW2, the switch SW3, and the switch SW4 to be in an interruption state, and controls the switch SW1 to be in a conductive state, so that discharging is performed from the power supply 12 to the first load 21 to atomize the aerosol source 22. Further, the power control unit controls the switch SW1, the switch SW2, and the switch SW4 to be in an interruption state and controls the switch SW3 to be in a conductive state, so that discharging is performed from the power supply 12 to the second load 31 to heat the flavor source 33.

In the case of the circuit example shown in Fig. 8, the power control unit controls the switch SW2 and the switch SW3 to be in an interruption state and controls the switch SW1 to be in a conductive state, so that discharging is performed from the power supply 12 to the first load 21 to atomize the aerosol source 22. Further, the power control unit controls the switch SW1 and the switch SW2 to be in an interruption state and controls the switch SW3 to be in a conductive state, so that discharging is performed from the power supply 12 to the second load 31 to heat the flavor source 33.

Accordingly, in the aerosol inhaler 1, the flavor source 33 can be heated by discharging to the second load 31. Therefore, if power discharged to the first load 21 is the same, the amount of the flavor component added to the aerosol can be increased by heating the flavor source 33 as compared with a case where the flavor source 33 is not heated.

A weight [mg] of an aerosol that is generated in the first cartridge 20 and passes through the flavor source 33 by one suction operation by the user is referred to as an aerosol weight $W_{aerosol}$. Power required to be supplied to the first load 21 for the generation of the aerosol is referred to as atomization power $P_{liquid}$. A time when the atomization power $P_{liquid}$ is supplied to the first load 21 for the generation of the aerosol is referred to as a supply time tsense. An upper limit value of the supply time $t_{sense}$ is the first default value $t_{upper}$ described above per suction. A weight [mg] of a flavor component contained in the flavor source 33 is referred to as a flavor component remaining amount $W_{capsule}$. Information on a temperature of the flavor source 33 is referred to as a temperature parameter $T_{capsule}$. A weight [mg] of a flavor component added to the aerosol that passes through the flavor source 33 by one suction operation by the user is referred to as an amount of a flavor component $W_{flavor}$. Specifically, the information on the temperature of the flavor source 33 is a temperature of the flavor source 33 or the second load 31 acquired based on an output of the temperature detection element T1 (or the temperature detection element T3).

It is experimentally found that the amount of the flavor component $W_{flavor}$ depends on the flavor component remaining amount $W_{capsule}$, the temperature parameter $T_{capsule}$, and the aerosol weight $W_{aerosol}$. Therefore, the amount of the flavor component $W_{flavor}$ can be modeled by the following equation (1).

$$W_{flavor} = \beta \times (W_{capsule} \times T_{capsule}) \times \gamma \times W_{aerosol} \quad (1)$$

The $\beta$ in Equation (1) is a coefficient indicating a ratio of how much of the flavor component contained in the flavor source 33 is added to an aerosol in one suction, and is experimentally obtained. The $\gamma$ in Equation (1) is a coefficient obtained experimentally. The temperature parameter $T_{capsule}$ and the flavor component remaining amount $W_{capsule}$ can fluctuate during a period in which one suction is performed, but in the model, the $\gamma$ is introduced in order to handle the temperature parameter $T_{capsule}$ and the flavor component remaining amount $W_{capsule}$ as constant values.

The flavor component remaining amount $W_{capsule}$ is decreased every time suction is performed. Therefore, the flavor component remaining amount $W_{capsule}$ is inversely proportional to suction times that are times when the suction is performed (in other words, the cumulative number of times of operations of discharging to the first load 21 for aerosol generation in response to the aerosol generation request). Further, the flavor component remaining amount $W_{capsule}$ decreases more as a time during which discharging to the first load 21 is performed to generate an aerosol in response to suction is longer. Therefore, the flavor component remaining amount $W_{capsule}$ is also inversely proportional to a cumulative value of the time during which the discharging to the first load 21 is performed to generate the aerosol in response to the suction (hereinafter, referred to as cumulative discharging time).

As can be seen from the model of Equation (1), when it is assumed that the amount of the aerosol $W_{aerosol}$ for each suction is controlled to be substantially constant, it is necessary to increase the temperature of the flavor source 33 according to a decrease in the flavor component remaining amount $W_{capsule}$ (in other words, an increase in the suction times or the cumulative discharging time) in order to stabilize the amount of the flavor component $W_{flavor}$.

Therefore, the power control unit of the MCU 50 increases a target temperature of the flavor source 33 (a target temperature $T_{cap\_target}$ described below) based on the suction times or the cumulative discharging time. Then, based on an output of the temperature detection element T1 (or the temperature detection element T3), the power control unit of the MCU 50 controls discharging for heating the flavor source 33 from the power supply 12 to the second load 31 such that the temperature of the flavor source 33 converges to the target temperature. Accordingly, it is possible to increase and stabilize the amount of the flavor component $W_{flavor}$. Specifically, the power control unit of the MCU 50 manages the target temperature according to a table stored in advance in the memory 50a. The table stores the suction times or the cumulative discharging time in association with the target temperature of the flavor source 33.

It is preferable that the target temperature of the flavor source 33 is increased in stages. Since the target temperature is increased in stages, frequent changes in the target temperature are prevented. Therefore, temperature control of the flavor source 33 can be controlled in a simple manner, and the control can be executed more stably.

It is more preferable that the target temperature of the flavor source 33 is increased in three or more stages. Since the target temperature is increased in three or more stages, it is possible to prevent the frequent changes in the target temperature and further stabilize the amount of the flavor component provided to the user.

It is preferable that the target temperature of the flavor source 33 is increased in stages in a temperature increment of 5°C or higher and 15°C or lower. In order to converge the temperature of the flavor source 33 to the target temperature, it is necessary to acquire the temperature of the flavor source 33 with a resolution finer than an increase width of the target temperature. The increase width of the target temperature is set to a value of 5°C or higher and 15°C or lower, so that an acquisition resolution of the temperature of the flavor source 33 can be set to 5°C or higher. Therefore, a cost required for acquiring the temperature of the flavor source 33 can be reduced.

The target temperature of the flavor source 33 may be increased in stages in a temperature increment of five times or more and fifteen times or less of the acquisition resolution of the temperature of the flavor source 33. Setting the acquisition resolution of the temperature of the flavor source 33 to lower than 1°C may lead to an increase in a cost. Since the increase width of the target temperature is set to the value of five times or more and fifteen times or less of the acquisition resolution, it is possible to achieve the increase width (the value of 5°C or higher and 15°C or lower) of the target temperature described above without increasing the cost required for acquiring the temperature of the flavor source 33. Further, frequent changes in the target temperature due to noise larger than the resolution can be prevented.

From a viewpoint of preventing the frequent changes in the target temperature, it is preferable that two or more values of the suction times are applied to at least one of a plurality of settable target temperatures. In other words, it is preferable that a plurality of pieces of information on the suction times are stored in association with at least one target temperature in the above table.

Accordingly, aerosol generation can be performed a plurality of times while setting one target temperature. Therefore, the frequent changes in the target temperature are prevented. An upper limit value of a time for discharging to the first load 21 in one suction is a first default value $t_{upper}$. Therefore, applying two or more values of the suction times to at least one of the plurality of settable target temperatures is synonymous with applying a width of the cumulative discharging time not less than two times the first default value $t_{upper}$ to at least one of the plurality of settable target temperatures.

Further, a lowest value among a plurality of target temperatures stored in the table may be a value included in a normal temperature (a temperature in a range of 5°C to 35°C defined by Japanese Industrial Standards). For example, immediately after the second cartridge 30 is replaced, the temperature of the flavor source 33 is likely to be a normal temperature. Further, immediately after the second cartridge 30 is replaced, a flavor component remaining amount of the flavor source 33 is a sufficient value, and a sufficient amount of a flavor component can be implemented without heating the flavor source 33. Therefore, since the lowest value of the target temperature is set to the normal temperature, in an initial stage where the suction times is small or the cumulative discharging time is short, it is not necessary to heat the flavor source 33, and power consumption can be reduced.

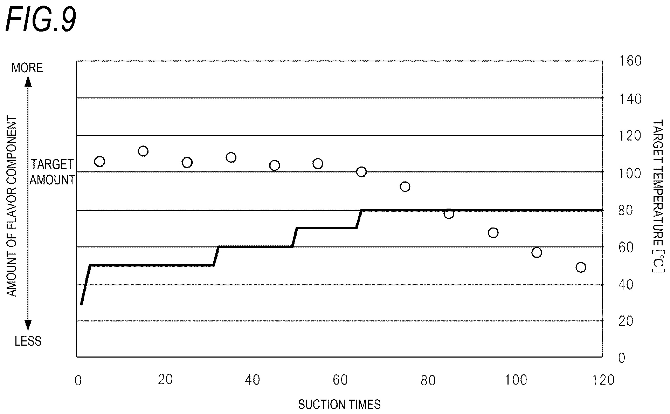

Fig. 9 is a diagram showing examples of results obtained by calculating the target temperature of the flavor source 33 such that the amount of the flavor component $W_{flavor}$ converges to the target amount, and examples of measurement results of the amount of the flavor component $W_{flavor}$ when discharging control to the second load 31 is performed for heating the flavor source 33 based on the result. Fig. 9 shows results when a total of one hundred and twenty suctions are performed, assuming that a time per suction is 2.4 seconds.

A horizontal axis in Fig. 9 indicates the suction times. A vertical axis on a right side of Fig. 9 indicates the target temperature of the flavor source 33. A vertical axis on a left side of Fig. 9 indicates the amount of the flavor component added to the aerosol by one suction. In Fig. 9, the amount of the flavor component when each N-th suction (N is a multiple of five) is performed is plotted as an experimental result. A thick solid line shown in Fig. 9 indicates a calculation result of the target temperature. The horizontal axis in Fig. 9 can be replaced with the cumulative discharging time by multiplying the suction times by 2.4 seconds.

According to a profile of the target temperature shown in Fig. 9, the amount of the flavor component for each suction can be substantially set to the target amount until a suction in the vicinity of eighty times. Accordingly, even for a user who repeats standard suction of 2.4 seconds, a large amount of a flavor component can be provided up to eighty suctions. Further, even when the suction is performed over eighty times, a larger amount of the flavor component can be provided as compared with a case where the flavor source is not heated.

Here, characteristics of the profile of the target temperature shown in Fig. 9 will be described.

Among periods for setting target temperatures (50°C, 60°C, 70°C, 80°C), a period for setting a highest target temperature (80°C) is the longest. This can be explained by a fact that, in a state where the suction times or the cumulative discharging time is large, the flavor component remaining amount of the flavor source 33 is small, and it is necessary to increase the temperature of the flavor source 33 in order to implement a stable amount of the flavor component. In addition, since the target temperature is prevented from reaching a high temperature due to frequent increases in the target temperature, it is possible to avoid an increase in power consumption and the flavor source 33 falling into an overheated state. Therefore, it is desirable that the power control unit of the MCU 50 increases the target temperature such that a period during which the highest target temperature is applied is the longest.

Among the periods for setting the target temperatures (50°C, 60°C, 70°C, 80°C), a period for setting a lowest target temperature (50°C) is the second longest after the period for setting the highest target temperature (80°C). This can be explained by a fact that, in a state where a value of the suction times or the cumulative discharging time is small, the flavor component remaining amount of the flavor source 33 is sufficiently present, and a stable amount of the flavor component can be implemented without increasing the temperature of the flavor source 33. According to the profile of Fig. 9, since the period for setting the lowest target temperature (50°C) is not the shortest (somewhat long), it is possible to suppress power required for heating the flavor source 33. Therefore, it is desirable that the power control unit of the MCU 50 increases the target temperature such that a period during which the lowest target temperature is applied is not the shortest.

As described above, two or more values of the suction times correspond to each target temperature. Further, a range (a width) of the cumulative discharging time corresponding to each target temperature is not less than two times the first default value $t_{upper}$. That is, the target temperature is not changed while the suction is performed a plurality of times. Accordingly, since the frequent changes in the target temperature are prevented, it is possible to reduce power consumption and simplify control.

In the above description, as a specific example, 50°C is given as the lowest target temperature. Alternatively, the lowest target temperature may be a value included in a normal temperature (a temperature in a range of 5°C to 35°C defined by Japanese Industrial Standards). Even in this case, it is preferable that the period for setting the lowest target temperature included in a normal temperature is the second longest and/or not the shortest (somewhat long) after the period for setting the highest target temperature (80°C).

In the above description, the target temperature is increased in three stages from the lowest target temperature (50°C) to the highest target temperature (80°C). Alternatively, the MCU 50 may increase the target temperature to the highest target temperature in more than three stages. An upper limit of the number of stages to go through is preferably, for example, five or less.

(Operations of Aerosol Inhaler)

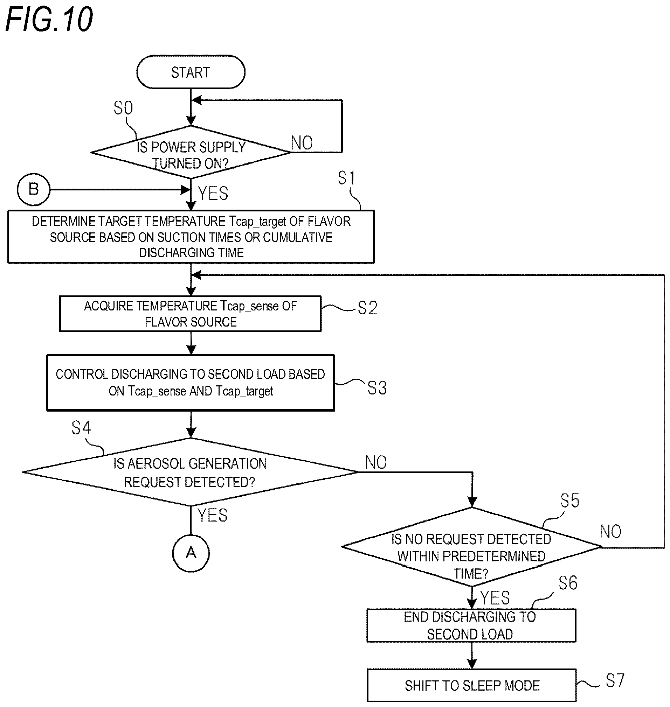

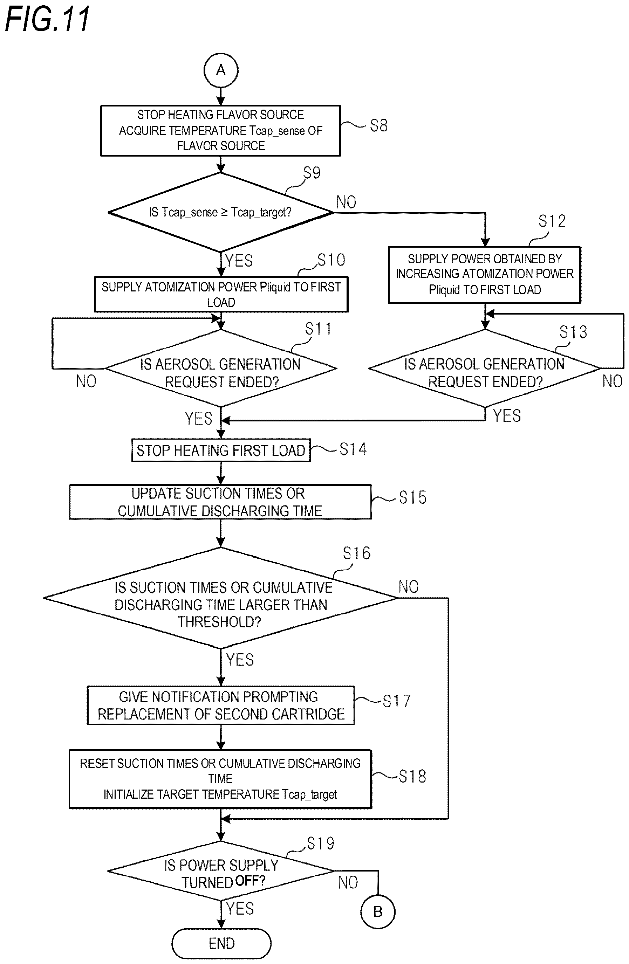

Figs. 10 and 11 are flowcharts for illustrating operations of the aerosol inhaler 1 of Fig. 1. When the power supply of the aerosol inhaler 1 is turned on by an operation of the operation unit 14 or the like (Step S0: YES), the MCU 50 determines (sets) the target temperature $T_{cap\_target}$ of the flavor source 33 based on the suction times or the cumulative discharging time stored in the memory 50a (Step S1).

Next, the MCU 50 acquires the current temperature $T_{cap\_sense}$ of the flavor source 33 based on an output of the temperature detection element T1 (or the temperature detection element T3) (Step S2).

Then, the MCU 50 controls discharging to the second load 31 for heating the flavor source 33 based on the temperature $T_{cap\_sense}$ and the target temperature $T_{cap\_target}$ (Step S3). Specifically, the MCU 50 supplies power to the second load 31 by proportional-integraldifferential (PID) control or ON/OFF control such that the temperature $T_{cap\_sense}$ converges to the target temperature $T_{cap\_target}$.

In the PID control, a difference between the temperature $T_{cap\_sense}$ and the target temperature $T_{cap\_target}$ is fed back, and based on the feedback result, power control is performed such that the temperature $T_{cap\_sense}$ converges to the target temperature $T_{cap\_target}$. According to the PID control, the temperature $T_{cap\_sense}$ can converge to the target temperature $T_{cap\_target}$ with high accuracy. The MCU 50 may use proportional (P) control or proportional-integral (PI) control instead of the PID control.

The ON/OFF control is control in which when the temperature $T_{cap\_sense}$ is lower than the target temperature $T_{cap\_target}$, power is supplied to the second load 31, and when the temperature $T_{cap\_sense}$ is equal to or higher than the target temperature $T_{cap\_target}$, the power supply to the second load 31 is stopped until the temperature $T_{cap\_sense}$ is lower than the target temperature $T_{cap\_target}$. According to the ON/OFF control, the temperature of the flavor source 33 can be increased faster than that in the PID control. Therefore, it is possible to increase a possibility that the temperature $T_{cap\_sense}$ reaches the target temperature $T_{cap\_target}$ at a stage before the aerosol generation request described later is detected. The target temperature $T_{cap\_target}$ may have hysteresis.

After Step S3, the MCU 50 determines whether there is the aerosol generation request (Step S4). When detecting no aerosol generation request (Step S4: NO), the MCU 50 determines, in Step S5, a length of a time during which the aerosol generation request is not made (hereinafter, referred to as non-operation time). Then, when the non-operation time reaches a predetermined time (Step S5: YES), the MCU 50 ends discharging to the second load 31 (Step S6), and shifts to a sleep mode in which power consumption is reduced (Step S7). When the non-operation time is less than a predetermined time (Step S5: NO), the MCU 50 shifts the processing to Step S2.

When the aerosol generation request is detected (Step S4: YES), the MCU 50 ends discharging to the second load 31 for heating the flavor source 33, and acquires the temperature $T_{cap\_sense}$ of the flavor source 33 at that time based on an output of the temperature detection element T1 (or the temperature detection element T3) (Step S8). Then, the MCU 50 determines whether the temperature $T_{cap\_sense}$ acquired in Step S8 is equal to or higher than the target temperature $T_{cap\_target}$ (Step S9).

When the temperature $T_{cap\_sense}$ is equal to or higher than the target temperature $T_{cap\_target}$ (Step S9: YES), the MCU 50 supplies the predetermined atomization power $P_{liquid}$ to the first load 21 to start heating the first load 21 (heating for atomizing the aerosol source 22) (Step S10). After the heating of the first load 21 is started in Step S10, the MCU 50 continues the heating when the aerosol generation request is not ended (Step S11: NO), and stops power supply to the first load 21 when the aerosol generation request is ended (Step S11: YES) (Step S14).

When the temperature $T_{cap\_sense}$ is lower than the target temperature $T_{cap\_target}$ (Step S9: NO), the MCU 50 supplies power obtained by increasing the atomization power $P_{liquid}$ by a predetermined amount to the first load 21 to start heating the first load 21 (Step S12). The increase in the power here is performed according to, for example, a table in which a temperature difference between the temperature $T_{cap\_sense}$ and the target temperature $T_{cap\_target}$ and a power increase amount are associated with each other. After the heating of the first load 21 is started in Step S12, the MCU 50 continues the heating when the aerosol generation request is not ended (Step S13: NO), and stops power supply to the first load 21 when the aerosol generation request is ended (Step S13: YES) (Step S14).

Accordingly, even when the temperature of the flavor source 33 does not reach the target temperature at a time point at which the aerosol generation request is made, by performing the processing of Step S12, an amount of a generated aerosol can be increased. As a result, a decrease in an amount of a flavor component added to an aerosol due to the temperature of the flavor source 33 being lower than the target temperature can be compensated for by an increase in the amount of the aerosol. Therefore, the amount of the flavor component added to the aerosol can converge to the target amount.

After Step S14, the MCU 50 updates the suction times or the cumulative discharging time stored in the memory 50a (Step S15).

Next, the MCU 50 determines whether the updated suction times or cumulative discharging time is larger than a threshold (Step S16). When the updated suction times or cumulative discharging time is equal to or smaller than the threshold (Step S16: NO), the MCU 50 shifts the processing to Step S19. When the updated suction times or cumulative discharging time is larger than the threshold (Step S16: YES), the MCU 50 causes the notification unit 45 to give a notification that prompts replacement of the second cartridge 30 (Step S17). Then, the MCU 50 resets the suction times or the cumulative discharging time to an initial value (= 0) and initializes the target temperature $T_{cap\_target}$ (Step S18). The initialization of the target temperature $T_{cap\_target}$ means excluding a target temperature $T_{cap\_target}$ at that time point stored in the memory 50a from a set value. As a specific example, when the MCU 50 uses the profile of the target temperature shown in Fig. 9, instead of the initialization, the lowest target temperature (50°C) may be set as the target temperature $T_{cap\_target}$. In that case, the processing of Step S1 performed immediately after the processing may be omitted.

After Step S18, the MCU 50 returns the processing to Step S1 if the power supply is not turned off (Step S19: NO) and ends the processing when the power supply is turned off (Step S19: YES).

(Effects of Embodiment)

As described above, according to the aerosol inhaler 1, the amount of the flavor component added to the aerosol generated in response to the aerosol generation request can be stabilized at a high value, and the commercial value can be increased.

In the flowcharts of Figs. 10 and 11, the target temperature is maintained at a value before the aerosol generation request is detected during a period from detection of the aerosol generation request to end of the aerosol generation request. When the cumulative discharging time is used to determine the target temperature, the cumulative discharging time is increased also during the period. However, as described above, control can be simplified by not changing the target temperature during the period. Further, power discharged from the power supply 12 can be suppressed, and deterioration of the power supply 12 can be avoided.

(First Modification of Aerosol inhaler)

On the assumption of a configuration that uses the cumulative discharging time to determine the target temperature, during the aerosol generation period in the flowcharts shown in Figs. 10 and 11 (a period from when the determination in Step S4 is YES to when the determination in Step S11 or Step S13 is YES), the MCU 50 may be configured to sequentially update the cumulative discharging time and change the target temperature of the flavor source 33 based on the updated cumulative discharging time.

Accordingly, by changing the target temperature during aerosol generation, an amount of a flavor component provided to a user for each suction of an aerosol and during suction can be further stabilized, and a commercial value can be further increased.

When the target temperature is increased during the aerosol generation period, not only discharging to the first load 21 but also discharging to the second load 31 is required. In such a case, it is preferable that the MCU 50 alternately performs discharging from the power supply 12 to the first load 21 for aerosol generation and discharging from the power supply 12 to the second load 31 for causing a temperature of the flavor source 33 to converge to an increased target temperature. In this way, it is possible to prevent the two loads from being discharged at the same time. Therefore, it is possible to discharge the two loads while avoiding deterioration of the power supply 12 due to discharging of a large current.

Alternatively, when the target temperature is increased during the aerosol generation period, the MCU 50 may shift the processing to Step S9 when the determination in Step S11 of Fig. 11 is NO and when the determination in Step S13 of Fig. 11 is NO. Accordingly, when the target temperature is increased, power discharged to the first load 21 in Step S12 can be increased. As the power discharged to the first load 21 increases, an amount of a generated aerosol increases. Therefore, an effect of increasing the temperature of the flavor source 33 can be obtained by the increased aerosol. As a result, even without discharging to the second load 31, the amount of the flavor component provided to the user for each suction of the aerosol and during suction can be stabilized, and control can be simplified. In such a case, the MCU 50 may shift the processing to Step S8 instead of Step S9. Accordingly, based on a comparison between the increased target temperature and a latest temperature of the flavor source 33, power discharged to the first load 21 in Step S12 can be increased. Therefore, the amount of the flavor component provided to the user for each suction of the aerosol and during suction can be further stabilized.

(Second Modification of Aerosol Inhaler)

In this modification, a configuration that uses the cumulative discharging time to determine the target temperature is assumed. In the configuration, a threshold is provided for the cumulative discharging time in order to change the target temperature. The threshold corresponds to a boundary value between a cumulative discharging time to which a first target temperature is applied and a cumulative discharging time to which a second target temperature is applied when the first target temperature and the second target temperature higher than the first target temperature can be set. For example, the MCU 50 may set the first target temperature if the cumulative discharging time is equal to or smaller than the threshold and set the second target temperature if the cumulative discharging time is larger than the threshold, but in this modification, the target temperature is switched as follows.

Specifically, the MCU 50 increases the target temperature when a difference between the cumulative discharging time and the threshold is less than the first default value $t_{upper}$, and maintains the target temperature when the difference is equal to or larger than the first default value $t_{upper}$.

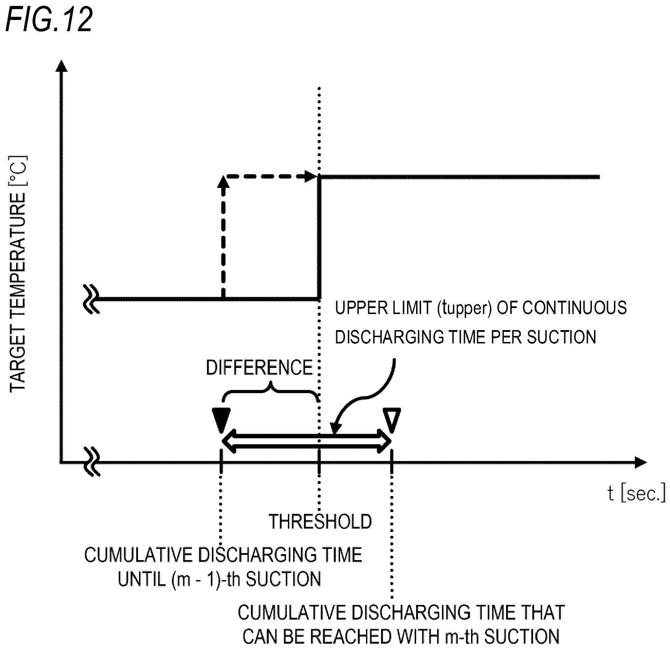

When the target temperature is changed using the threshold as described above, for example, as shown in Fig. 12, a difference between the cumulative discharging time and the threshold after (m - 1)-th suction is performed may be less than the first default value $t_{upper}$ that is an upper limit of a time when the first load 21 can be discharged per suction. Even in this situation, the MCU 50 increases the target temperature as indicated by dotted lines in Fig. 12 when the difference between the cumulative discharging time and the threshold is less than the first default value $t_{upper}$, and maintains the target temperature as indicated by a solid line in Fig. 12 when the difference is equal to or larger than the first default value $t_{upper}$. Accordingly, it is possible to set the target temperature according to a cumulative discharging time that can be reached at a time of next aerosol generation. As a result, an amount of a flavor component can be stabilized.

In the above embodiment and modifications, the configuration is provided in which the first cartridge 20 is detachable from the power supply unit 10, but a configuration may be provided in which the first cartridge 20 is integrated with the power supply unit 10.

In the above embodiment and modifications, the first load 21 and the second load 31 are heaters that generate heat by power discharged from the power supply 12, but the first load 21 and the second load 31 may be Peltier elements that can perform both heat generation and cooling by the power discharged from the power supply 12. If the first load 21 and the second load 31 are configured in this way, a degree of freedom of control related to the temperature of the aerosol source 22 and the temperature of the flavor source 33 is increased, so that the unit flavor amount can be more highly controlled.

Further, the first load 21 may be configured with an element that can atomize the aerosol source 22 without heating the aerosol source 22 by ultrasonic waves or the like. Further, the second load 31 may be configured with an element that can change the amount of the flavor component added to the aerosol by the flavor source 33 without heating the flavor source 33 by the ultrasonic waves or the like.

An element that can be used for the first load 21 is not limited to the heater, the Peltier element, and the ultrasonic wave element described above, and various elements or combinations thereof can be used as long as the element can atomize the aerosol source 22 by consuming the power supplied from the power supply 12. Similarly, an element that can be used for the second load 31 is not limited to the heater, the Peltier element, and the ultrasonic wave element described above, and various elements or combinations thereof can be used as long as the element can change the amount of the flavor component added to the aerosol by consuming the power supplied from the power supply 12.

In the above description, the MCU 50 controls discharging from the power supply 12 to the first load 21 and the second load 31 such that the amount of the flavor component $W_{flavor}$ converges to the target amount. The target amount is not limited to a specific value and may be a range having a certain width.

There is described herein a power supply unit (a power supply unit 10) for an aerosol inhaler (an aerosol inhaler 1) that causes an aerosol generated from an aerosol source (an aerosol source 22) heated by a first load (a first load 21) to pass through a flavor source (a flavor source 33) heated by a second load (a second load 31) to add a flavor component of the flavor source to the aerosol, the power supply unit including:

> a power supply (a power supply 12) configured to be dischargeable to the first load and the second load; and
> a processing device (an MCU 50),
> in which the processing device is configured to control discharging from the power supply to the first load in response to a signal from a first sensor (an intake sensor 15 or an operation unit 14) configured to output the signal indicating an aerosol generation request,
> in which the processing device is configured to control discharging from the power supply to the second load such that a temperature of the flavor source converges to a target temperature based on an output of a second sensor (a temperature detection element T1 or a temperature detection element T3) configured to output information on a temperature of the flavor source, and
> in which the processing device is configured to increase the target temperature based on a cumulative number of times (suction times) of discharging to the first load in response to the signal or a cumulative time (a cumulative discharging time) of discharging to the first load in response to the signal.

This power supply unit, which does not incorporate all the essential technical features of the present invention but is useful for understanding the invention, makes it possible, since the target temperature of the flavor source is increased based on a parameter (the cumulative times or the cumulative time) indicating consumption of the flavor source, to stabilize an amount of a flavor component provided to a user for each suction of the aerosol while performing simple control. As a result, a commercial value of the aerosol inhaler can be increased.

According to the power supply unit, if the processing device is configured to increase the target temperature in stages based on the cumulative number of times or the cumulative time, frequent changes in the target temperature are prevented by gradually increasing the target temperature. Therefore, it is possible to execute control more stably while performing simple control.

The power supply unit makes it possible, if the processing device is configured to increase the target temperature in three or more stages based on the cumulative number of times or the cumulative time, to prevent the frequent changes in the target temperature and further stabilize the amount of the flavor component provided to the user, since the target temperature is gradually increased at an appropriate frequency.

The power supply unit makes it possible, if the processing device is configured to increase the target temperature in stages in a temperature increment of 5°C or higher and 15°C or lower based on the cumulative times or the cumulative time, to prevent an increase in power consumption and deterioration of the power supply due to the frequent changes in the target temperature, since the target temperature is gradually increased at an appropriate value.

According to the power supply unit, if the processing device is configured to acquire a temperature of the flavor source with a predetermined resolution based on an output of the second sensor, and the processing device is configured to increase the target temperature in stages in a temperature increment of five times or more and fifteen times or less of the resolution based on the cumulative number of times or the cumulative time, the target temperature is gradually increased by a value sufficiently larger than the acquisition resolution of the temperature of the flavor source. Therefore, it is unnecessary to devise ways to reduce the resolution, and a manufacturing cost of the aerosol inhaler can be reduced. Further, the frequent changes in the target temperature due to noise larger than the resolution can be prevented.

In a power supply unit according to the invention, the processing device is configured to increase the target temperature such that a period during which the highest target temperature is applied is the longest. Therefore, it is possible to stabilize the amount of the flavor component by preventing the amount of the flavor component provided to the user from decreasing. Further, since the target temperature is prevented from reaching a high temperature due to frequent increases in the target temperature, an increase in power consumption and the flavor source falling into an overheated state can be avoided.

According to the power supply unit, if the processing device is configured to increase the target temperature such that a period during which the lowest target temperature is applied is not the shortest, power consumption required for heating the flavor source can be suppressed.

According to the power supply unit, if the lowest target temperature is included in a normal temperature, since the flavor source can also be prevented from being heated at the start of use of the aerosol inhaler, the power consumption required for heating the flavor source can be further suppressed.

According to the power supply unit, if the processing device is configured to increase the target temperature based on the cumulative time, to control discharging from the power supply to the first load such that a time for discharging to the first load per aerosol generation request is equal to or smaller than a predetermined time (a first default value $t_{upper}$), and to apply a width of the cumulative time not less than two times the predetermined time to at least one value of the target temperature, aerosol generation can be performed a plurality of times at one target temperature. Therefore, the frequent changes in the target temperature are prevented. Therefore, it is possible to execute control more stably while performing simple control.

According to the power supply unit, if the processing device is configured to increase the target temperature based on the cumulative times, and to apply two or more values of the cumulative number of times to at least one value of the target temperature, the aerosol generation can be performed the plurality of times at one target temperature. Therefore, the frequent changes in the target temperature are prevented. Therefore, it is possible to execute control more stably while performing simple control.

According to the power supply unit, if the processing device is configured to increase the target temperature based on the cumulative time and to maintain the target temperature while discharging is performed from the power supply to the first load, the target temperature is maintained during the aerosol generation. Therefore, it is possible to prevent an increase in power discharged to the second load during the aerosol generation. Therefore, sufficient power can be discharged to the first load while avoiding deterioration of the power supply due to discharging of a large current. Further, control can be simplified.

According to the power supply unit, if the processing device is configured to increase the target temperature based on the cumulative time and to also increase the target temperature according to the cumulative time while discharging is performed from the power supply to the first load, the target temperature can be changed during the aerosol generation. Therefore, the amount of the flavor component provided to the user for each suction of the aerosol and during suction can be stabilized, and the commercial value can be further increased.

The power supply unit makes it possible, if the processing device alternately performs discharging from the power supply to the first load and discharging from the power supply to the second load for causing a temperature of the flavor source to converge to the increased target temperature when the target temperature is increased while discharging is performed from the power supply to the first load, to prevent the two loads from being discharged at the same time. Therefore, it is possible to sufficiently discharge the two loads in order to stabilize the amount of the flavor component provided to the user, while avoiding the deterioration of the power supply due to the discharging of the large current.

According to the power supply unit, if the processing device increases power discharged from the power supply to the first load when the target temperature is increased while discharging is performed from the power supply to the first load, the power discharged to the first load is increased when the target temperature is changed during the aerosol generation. Therefore, even when the flavor source does not reach the target temperature, the amount of the flavor component added to the aerosol can be increased by increasing the aerosol. As a result, the amount of the flavor component provided to the user for each suction of the aerosol and during suction can be stabilized, and the commercial value can be further increased.

According to the power supply unit, if the processing device is configured to control discharging of power from the power supply to the first load such that a time for discharging to the first load per aerosol generation request is equal to or smaller than a predetermined time (a first default value $t_{upper}$), and the processing device increases the target temperature when a difference between the cumulative time and a time threshold before discharging to the first load in response to the signal is less than the predetermined time, and maintains the target temperature when the difference is equal to or larger than the predetermined time, then if the amount of the flavor component contained in the aerosol decreases at next suction, the target temperature of the flavor source is increased in advance. Therefore, the amount of the flavor component added to the aerosol for each suction and during suction can be stabilized.

An aerosol inhaler not according to the invention can be provided with a power supply unit for an aerosol inhaler that causes an aerosol generated from an aerosol source for which at least heating of the heating and cooling is performed by a first load to pass through a flavor source for which at least heating of the heating and cooling is performed by a second load to add a flavor component of the flavor source to the aerosol, the power supply unit including:

a power supply configured to be dischargeable to the first load and the second load; and
a processing device,
in which the processing device is configured to control discharging from the power supply to the first load in response to a signal from a first sensor configured to output the signal indicating an aerosol generation request,
in which the processing device is configured to control discharging from the power supply to the second load such that a temperature of the flavor source converges to a target temperature based on an output of a second sensor configured to output information on a temperature of the flavor source, and
in which the processing device is configured to increase the target temperature based on a cumulative number of times of discharging to the first load in response to the signal or a cumulative time of discharging to the first load in response to the signal.

An aerosol inhaler not according to the invention includes:

the power supply unit described above;
the aerosol source;
the flavor source;
the first load;
the second load;
the first sensor; and
the second sensor.

**Claims**

1.   A power supply unit (10) for an aerosol inhaler (10) that causes an aerosol generated from an aerosol source (22) heated by a first load (21) to pass through a flavor source (33) heated by a second load (31) to add a flavor component of the flavor source to the aerosol, the power supply unit comprising:

a power supply (12) configured to be dischargeable to the first load and the second load; and
a processing device (50),
wherein the processing device is configured to control discharging from the power supply to the first load in response to a signal from a first sensor (14; 15) configured to output the signal indicating an aerosol generation

EP 3 871 518 B1

request,
wherein the processing device is configured to control discharging from the power supply to the second load such that a temperature of the flavor source converges to a target temperature based on an output of a second sensor (T1; T3) configured to output information on a temperature of the flavor source, and
wherein the processing device is configured to increase the target temperature such that a period during which the highest target temperature is applied is the longest, based on a cumulative number of times of discharging to the first load in response to the signal or a cumulative time of discharging to the first load in response to the signal.

2. The power supply unit (10) according to claim 1,
wherein the processing device (50) is configured to increase the target temperature in stages based on the cumulative number of times or the cumulative time.

3. The power supply unit (10) according to claim 2,
wherein the processing device (50) is configured to increase the target temperature in three or more stages based on the cumulative number of times or the cumulative time.

4. The power supply unit (10) according to claim 2 or 3,
wherein the processing device (50) is configured to increase the target temperature in stages in a temperature increment of 5°C or higher and 15°C or lower based on the cumulative number of times or the cumulative time.

5. The power supply unit (10) according to claim 2 or 3,

wherein the processing device (50) is configured to acquire a temperature of the flavor source (33) with a predetermined resolution based on an output of the second sensor (T1; T3), and
wherein the processing device (50) is configured to increase the target temperature in stages in a temperature increment of five times or more and fifteen times or less of the resolution based on the cumulative number of times or the cumulative time.

6. The power supply unit (10) according to claim 1,
wherein the processing device (50) is configured to increase the target temperature such that a period during which the lowest target temperature is applied is not the shortest.

7. The power supply unit (10) according to claim 1,
wherein the lowest target temperature is included in a normal temperature.

8. The power supply unit (10) according to claim 1,
wherein the processing device (50) is configured to increase the target temperature based on the cumulative time, to control discharging from the power supply (12) to the first load (21) such that a time for discharging to the first load per aerosol generation request is equal to or smaller than a predetermined time, and to apply a width of the cumulative time not less than two times the predetermined time to at least one value of the target temperature.

9. The power supply unit (10) according to claim 1,
wherein the processing device (50) is configured to increase the target temperature based on the cumulative number of times, and to apply two or more values of the cumulative number of times to at least one value of the target temperature.

10. The power supply unit (10) according to claim 1,
wherein the processing device (50) is configured to increase the target temperature based on the cumulative time and to maintain the target temperature while discharging is performed from the power supply (12) to the first load (21).

11. The power supply unit (10) according to claim 1,
wherein the processing device (50) is configured to increase the target temperature based on the cumulative time and to also increase the target temperature according to the cumulative time while discharging is performed from the power supply (12) to the first load (21).

12. The power supply unit (10) according to claim 11,
wherein the processing device (50) is configured to, when the target temperature is increased while discharging is performed from the power supply (12) to the first load (21), alternately perform discharging from the power supply to the first load and discharging from the power supply to the second load (31) for causing a temperature of the flavor source (33) to converge to the increased target temperature.

**13.** The power supply unit (10) according to claim 11,
wherein the processing device (50) is configured to, when the target temperature is increased while discharging is performed from the power supply (12) to the first load (21), increase power discharged from the power supply to the first load.

**14.** The power supply unit (10) according to claim 1,

wherein the processing device (50) is configured to control discharging of power from the power supply (12) to the first load (21) such that a time for discharging to the first load per aerosol generation request is equal to or smaller than a predetermined time, and

wherein the processing device (50) is configured to increase the target temperature when a difference between the cumulative time and a time threshold before discharging to the first load in response to the signal is less than the predetermined time, and to maintain the target temperature when the difference is equal to or larger than the predetermined time.

**15.** An aerosol inhaler (1) comprising the power supply unit (10) according to any of claims 1 to 14, the aerosol source (22); the flavor source (33); the first load (21); the second load (31); the first sensor (14;15); and the second sensor (T1; T3).

**Patentansprüche**

**1.** Stromversorgungseinheit (10) für einen Aerosolinhalator (10), die ein Aerosol, das von einer Aerosolquelle (22) erzeugt wird, die von einer ersten Last (21) erhitzt wird, veranlasst, eine Aromaquelle (33) zu passieren, die von einer zweiten Last (31) erhitzt wird, um eine Aromakomponente der Aromaquelle zum Aerosol hinzuzufügen, wobei die Stromversorgungseinheit umfasst:

eine Stromversorgung (12), die dazu ausgelegt ist, zu der ersten Last und der zweiten Last entladen werden zu können; und
eine Verarbeitungsvorrichtung (50),
wobei die Verarbeitungsvorrichtung dazu ausgelegt ist, Entladen von der Stromversorgung an die erste Last in Reaktion auf ein Signal von einem ersten Sensor (14; 15), der dazu ausgelegt ist, das Signal auszugeben, das eine Aerosolerzeugungsanforderung angibt, zu steuern,
wobei die Verarbeitungsvorrichtung dazu ausgelegt ist, Entladen von der Stromversorgung an die zweite Last auf Basis einer Ausgabe eines zweiten Sensors (T1; T3), der dazu ausgelegt ist, Informationen über eine Temperatur der Aromaquelle auszugeben, derart zu steuern, dass eine Temperatur der Aromaquelle zu einer Zieltemperatur konvergiert, und
wobei die Verarbeitungsvorrichtung dazu ausgelegt ist, die Zieltemperatur auf Basis einer kumulativen Anzahl vom Entladen an die erste Last in Reaktion auf das Signal oder einer kumulativen Zeit vom Entladen an die erste Last in Reaktion auf das Signal, derart anzuheben, dass eine Dauer, während derer die höchste Zieltemperatur angewendet wird, die längste ist.

**2.** Stromversorgungseinheit (10) nach Anspruch 1,
wobei die Verarbeitungsvorrichtung (50) dazu ausgelegt ist, die Zieltemperatur stufenweise auf Basis der kumulativen Anzahl oder der kumulativen Zeit anzuheben.

**3.** Stromversorgungseinheit (10) nach Anspruch 2,
wobei die Verarbeitungsvorrichtung (50) dazu ausgelegt ist, die Zieltemperatur in drei oder mehr Stufen auf Basis der kumulativen Anzahl oder der kumulativen Zeit anzuheben.

**4.** Stromversorgungseinheit (10) nach Anspruch 2 oder 3,
wobei die Verarbeitungsvorrichtung (50) dazu ausgelegt ist, die Zieltemperatur stufenweise in einer Temperaturerhöhung von 5°C oder höher und 15°C oder weniger auf Basis der kumulativen Anzahl oder der kumulativen Zeit anzuheben.

**5.** Stromversorgungseinheit (10) nach Anspruch 2 oder 3,

wobei die Verarbeitungsvorrichtung (50) dazu ausgelegt ist, eine Temperatur der Aromaquelle (33) mit einer vorgegebenen Auflösung auf Basis einer Ausgabe des zweiten Sensors (T1; T3) zu beschaffen, und
wobei die Verarbeitungsvorrichtung (50) dazu ausgelegt ist, die Zieltemperatur stufenweise in einer Temperaturerhöhung von fünfmal oder mehr und fünfzehnmal oder weniger der Auflösung auf Basis der kumulativen Anzahl oder der kumulativen Zeit anzuheben.

**6.** Stromversorgungseinheit (10) nach Anspruch 1,
wobei die Verarbeitungsvorrichtung (50) dazu ausgelegt ist, die Zieltemperatur derart anzuheben, dass eine Dauer, während derer die niedrigste Zieltemperatur angewendet wird, nicht die kürzeste ist.

**7.** Stromversorgungseinheit (10) nach Anspruch 1,
wobei die niedrigste Zieltemperatur in einer Normaltemperatur beinhaltet ist.

**8.** Stromversorgungseinheit (10) nach Anspruch 1,
wobei die Verarbeitungsvorrichtung (50) dazu ausgelegt ist, die Zieltemperatur auf Basis der kumulativen Zeit anzuheben, um Entladen von der Stromversorgung (12) an die erste Last (21) derart zu steuern, dass eine Zeit zum Entladen an die erste Last pro Aerosolerzeugungsanforderung gleich oder kleiner als eine vorgegebene Zeit ist, und eine Weite der kumulativen Zeit von nicht weniger als zweimal der vorgegebenen Zeit auf zumindest einen Wert der Zieltemperatur anzuwenden.

**9.** Stromversorgungseinheit (10) nach Anspruch 1,
wobei die Verarbeitungsvorrichtung (50) dazu ausgelegt ist, die Zieltemperatur auf Basis der kumulativen Anzahl anzuheben und zwei oder mehr Werte der kumulativen Anzahl auf zumindest einen Wert der Zieltemperatur anzuwenden.

**10.** Stromversorgungseinheit (10) nach Anspruch 1,
wobei die Verarbeitungsvorrichtung (50) dazu ausgelegt ist, die Zieltemperatur auf Basis der kumulativen Zeit anzuheben und die Zieltemperatur beizubehalten, während Entladen von der Stromversorgung (12) an die erste Last (21) durchgeführt wird.

**11.** Stromversorgungseinheit (10) nach Anspruch 1,
wobei die Verarbeitungsvorrichtung (50) dazu ausgelegt ist, die Zieltemperatur auf Basis der kumulativen Zeit anzuheben und auch die Zieltemperatur gemäß der kumulativen Zeit anzuheben, während Entladen von der Stromversorgung (12) an die erste Last (21) durchgeführt wird.

**12.** Stromversorgungseinheit (10) nach Anspruch 11,
wobei die Verarbeitungsvorrichtung (50) dazu ausgelegt ist, wenn die Zieltemperatur angehoben wird, während Entladen von der Stromversorgung (12) an die erste Last (21) durchgeführt wird, abwechselnd Entladen von der Stromversorgung an die erste Last und Entladen von der Stromversorgung an die zweite Last (31) durchzuführen, um eine Temperatur der Aromaquelle (33) zu veranlassen, zu der angehobenen Zieltemperatur zu konvergieren.

**13.** Stromversorgungseinheit (10) nach Anspruch 11,
wobei die Verarbeitungsvorrichtung (50) dazu ausgelegt ist, wenn die Zieltemperatur angehoben wird, während Entladen von der Stromversorgung (12) an die erste Last (21) durchgeführt wird, Strom anzuheben, der von der Stromversorgung an die erste Last entladen wird.

**14.** Stromversorgungseinheit (10) nach Anspruch 1,

wobei die Verarbeitungsvorrichtung (50) dazu ausgelegt ist, Entladen von Strom der Stromversorgung (12) an die erste Last (21) derart zu steuern, dass eine Zeit zum Entladen an die erste Last pro Aerosolerzeugungsanforderung gleich oder kürzer als eine vorgegebene Zeit ist, und
wobei die Verarbeitungsvorrichtung (50) dazu ausgelegt ist, die Zieltemperatur anzuheben, wenn eine Differenz zwischen der kumulativen Zeit und einer Zeitschwelle vor Entladen an die erste Last in Reaktion auf das Signal geringer als die vorgegebene Zeit ist, und die Zieltemperatur beizubehalten, wenn die Differenz gleich oder größer als die vorgegebene Zeit ist.

**15.** Aerosolinhalator (1), der die Stromversorgungseinheit (10) nach einem der Ansprüche 1 bis 14, die Aerosolquelle (22), die Aromaquelle (33); die erste Last (21); die zweite Last (31); den ersten Sensor (14; 15); und den zweiten Sensor (T1; T3) umfasst.

**Revendications**

**1.** Unité d'alimentation électrique (10) pour un inhalateur aérosol (10) qui amène un aérosol généré à partir d'une source d'aérosol (22) chauffée par une première charge (21) à passer à travers une source d'arôme (33) chauffée par une seconde charge (31) pour ajouter un composant d'arôme de la source d'arôme à l'aérosol, l'unité d'alimentation électrique comprenant :

une alimentation électrique (12) configurée pour être déchargeable sur la première charge et la seconde charge ; et
un dispositif de traitement (50),
dans laquelle le dispositif de traitement est configuré pour commander la décharge depuis l'alimentation électrique sur la première charge en réponse à un signal provenant d'un premier capteur (14 ; 15) configuré pour délivrer en sortie le signal indiquant une demande de génération d'aérosol,
dans laquelle le dispositif de traitement est configuré pour commander la décharge depuis l'alimentation électrique sur la seconde charge de sorte qu'une température de la source d'arôme converge vers une température cible sur la base d'une sortie d'un second capteur (T1 ; T3) configuré pour délivrer en sortie des informations sur une température de la source d'arôme, et
dans laquelle le dispositif de traitement est configuré pour augmenter la température cible de sorte qu'une période durant laquelle la température cible la plus haute est appliquée soit la plus longue, sur la base d'un nombre de fois cumulatif de décharge sur la première charge en réponse au signal ou d'une durée cumulative de décharge sur la première charge en réponse au signal.

**2.** Unité d'alimentation électrique (10) selon la revendication 1,
dans laquelle le dispositif de traitement (50) est configuré pour augmenter la température cible en étapes sur la base du nombre de fois cumulatif ou de la durée cumulative.

**3.** Unité d'alimentation électrique (10) selon la revendication 2,
dans laquelle le dispositif de traitement (50) est configuré pour augmenter la température cible en trois étapes ou plus sur la base du nombre de fois cumulatif ou de la durée cumulative.

**4.** Unité d'alimentation électrique (10) selon la revendication 2 ou 3,
dans laquelle le dispositif de traitement (50) est configuré pour augmenter la température cible en étapes avec un incrément de température de 5 °C ou plus et de 15 °C ou moins sur la base du nombre de fois cumulatif ou de la durée cumulative.

**5.** Unité d'alimentation électrique (10) selon la revendication 2 ou 3,

dans laquelle le dispositif de traitement (50) est configuré pour obtenir une température de la source d'arôme (33) avec une résolution prédéterminée sur la base d'une sortie du second capteur (T1 ; T3), et
dans laquelle le dispositif de traitement (50) est configuré pour augmenter la température cible en étapes avec un incrément de température de cinq fois ou plus et de quinze fois ou moins la résolution sur la base du nombre de fois cumulatif ou de la durée cumulative.

**6.** Unité d'alimentation électrique (10) selon la revendication 1,
dans laquelle le dispositif de traitement (50) est configuré pour augmenter la température cible de sorte qu'une période durant laquelle la température cible la plus faible est appliquée ne soit pas la plus courte.

**7.** Unité d'alimentation électrique (10) selon la revendication 1,
dans laquelle la température cible la plus faible est incluse dans une température normale.

**8.** Unité d'alimentation électrique (10) selon la revendication 1,
dans laquelle le dispositif de traitement (50) est configuré pour augmenter la température cible sur la base de la durée cumulative, pour commander la décharge depuis l'alimentation électrique (12) sur la première charge (21) de sorte qu'une durée de décharge sur la première charge par demande de génération d'aérosol soit égale ou inférieure à une durée prédéterminée, et pour appliquer une largeur de la durée cumulative non inférieure à deux fois la durée prédéterminée à au moins une valeur de la température cible.

9. Unité d'alimentation électrique (10) selon la revendication 1,
dans laquelle le dispositif de traitement (50) est configuré pour augmenter la température cible sur la base du nombre de fois cumulatif, et pour appliquer deux valeurs ou plus du nombre de fois cumulatif à au moins une valeur de la température cible.

10. Unité d'alimentation électrique (10) selon la revendication 1,
dans laquelle le dispositif de traitement (50) est configuré pour augmenter la température cible sur la base de la durée cumulative et pour maintenir la température cible tandis que la décharge est effectuée depuis l'alimentation électrique (12) sur la première charge (21).

11. Unité d'alimentation électrique (10) selon la revendication 1,
dans laquelle le dispositif de traitement (50) est configuré pour augmenter la température cible sur la base de la durée cumulative et également pour augmenter la température cible selon la durée cumulative tandis que la décharge est effectuée depuis l'alimentation électrique (12) sur la première charge (21).

12. Unité d'alimentation électrique (10) selon la revendication 11,
dans laquelle le dispositif de traitement (50) est configuré pour, lorsque la température cible est augmentée tandis que la décharge est effectuée depuis l'alimentation électrique (12) sur la première charge (21), effectuer alternativement la décharge depuis l'alimentation électrique sur la première charge et la décharge depuis l'alimentation électrique sur la seconde charge (31) pour amener une température de la source d'arôme (33) à converger vers la température cible augmentée.

13. Unité d'alimentation électrique (10) selon la revendication 11,
dans laquelle le dispositif de traitement (50) est configuré pour, lorsque la température cible est augmentée tandis que la décharge est effectuée depuis l'alimentation électrique (12) sur la première charge (21), augmenter l'alimentation déchargée depuis l'alimentation électrique sur la première charge.

14. Unité d'alimentation électrique (10) selon la revendication 1,

dans laquelle le dispositif de traitement (50) est configuré pour commander la décharge d'alimentation depuis l'alimentation électrique (12) sur la première charge (21) de sorte qu'une durée de décharge sur la première charge par demande de génération d'aérosol soit égale ou inférieure à une durée prédéterminée, et
dans laquelle le dispositif de traitement (50) est configuré pour augmenter la température cible lorsqu'une différence entre la durée cumulative et un seuil de durée avant la décharge sur la première charge en réponse au signal est inférieure à la durée prédéterminée, et pour maintenir la température cible lorsque la différence est égale ou supérieure à la durée prédéterminée.

15. Inhalateur aérosol (1) comprenant l'unité d'alimentation électrique (10) selon l'une quelconque des revendications 1 à 14, la source d'aérosol (22) ; la source d'arôme (33) ; la première charge (21) ; la seconde charge (31) ; le premier capteur (14; 15) ; et le second capteur (T1 ; T3).

*FIG.1*

*FIG.2*

**FIG.3**

**FIG.4**

FIG.5

## FIG.6

# FIG.7

FIG.8

## FIG.9

## FIG.10

START

S0

IS POWER SUPPLY
TURNED ON? — NO

B → YES

S1

DETERMINE TARGET TEMPERATURE Tcap_target OF FLAVOR
SOURCE BASED ON SUCTION TIMES OR CUMULATIVE
DISCHARGING TIME

ACQUIRE TEMPERATURE Tcap_sense OF
FLAVOR SOURCE — S2

CONTROL DISCHARGING TO SECOND LOAD BASED
ON Tcap_sense AND Tcap_target — S3

S4

IS AEROSOL GENERATION
REQUEST DETECTED? — NO

YES

A

S5

IS NO REQUEST DETECTED
WITHIN PREDETERMINED
TIME? — NO

YES — S6

END DISCHARGING TO
SECOND LOAD

SHIFT TO SLEEP MODE — S7

# FIG.11

(A)

STOP HEATING FLAVOR SOURCE
ACQUIRE TEMPERATURE Tcap_sense OF
FLAVOR SOURCE — S8

S9
IS Tcap_sense ≥ Tcap_target? — NO

YES — S10
SUPPLY ATOMIZATION POWER Pliquid TO FIRST
LOAD

S12
SUPPLY POWER OBTAINED BY
INCREASING ATOMIZATION POWER
Pliquid TO FIRST LOAD

S11
IS AEROSOL GENERATION
REQUEST ENDED? — NO

S13
IS AEROSOL GENERATION
REQUEST ENDED? — NO

YES

YES

STOP HEATING FIRST LOAD — S14

UPDATE SUCTION TIMES OR
CUMULATIVE DISCHARGING TIME — S15

S16
IS SUCTION TIMES OR CUMULATIVE
DISCHARGING TIME LARGER THAN
THRESHOLD? — NO

YES

GIVE NOTIFICATION PROMPTING
REPLACEMENT OF SECOND CARTRIDGE — S17

RESET SUCTION TIMES OR CUMULATIVE DISCHARGING
TIME
INITIALIZE TARGET TEMPERATURE Tcap_target — S18

S19
IS POWER SUPPLY
TURNED OFF? — NO — (B)

YES

END

## FIG.12

TARGET TEMPERATURE [°C]

DIFFERENCE

UPPER LIMIT (tupper) OF CONTINUOUS
DISCHARGING TIME PER SUCTION

THRESHOLD

t [sec.]

CUMULATIVE DISCHARGING TIME
UNTIL (m - 1)-th SUCTION

CUMULATIVE DISCHARGING TIME THAT
CAN BE REACHED WITH m-th SUCTION

**EP 3 871 518 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017511703 T
- WO 2019017654 A
- WO 2018020619 A
- WO 2020027448 A1
- EP 3066942 A1